# EUROPEAN PATENT SPECIFICATION

(11) **EP 1 865 075 B1**
(45) Date of publication and mention of the grant of the patent: **12.09.2012**
(21) Application number: 07109428.8
(22) Date of filing: 01.06.2007
(51) Int. Cl.: C12Q 1/68

(54) **A method of amplifying nucleic acid from blood**
Verfahren zur Amplifikation von Nukleinsäuren aus Vollblut
Procédé d'amplification d'acides nucléiques à partir du sang

(30) Priority: 05.06.2006 KR 20060050478
(43) Date of publication of application: 12.12.2007
(73) Proprietor: Samsung Electronics Co., Ltd., Suwon-si, Gyeonggi-do 442-743 (KR)
(72) Inventor: Lee, Hun-joo, Gyeonggi-do (KR); Jeong, Sung-young, Gyeonggi-do (KR); Kim, Joon-ho, Gyeonggi-do (KR); Hwang, Kyu-youn, Gyeonggi-do (KR)
(74) Representative: Grünecker, Kinkeldey, Stockmair & Schwanhäusser

(56) References cited:
- EP-A- 1 672 059
- WO-A-2005/083078
- US-A- 4 306 556
- US-A- 5 437 774
- US-B1- 6 284 117
- JOON-HO KIM ET AL: "A disposable DNA sample preparation microfluidic chip for nucleic acid probe assay" PROCEEDINGS OF THE IEEE 15TH. ANNUAL INTERNATIONAL CONFERENCE ON MICROELECTRO MECHANICAL SYSTEMS. MEMS 2002. LAS VEGAS, NV, JAN. 20 - 24, 2002, IEEE INTERNATIONAL MICRO ELECTRO MECHANICAL SYSTEMS CONFERENCE, NEW YORK, NY : IEEE, US, vol. CONF. 15, 2002, pages 133-136, XP010577613 ISBN: 0-7803-7185-2
- SATO T ET AL: "BACTERICIDAL EFFECT OF AN ELECTRODIALYSIS SYSTEM ON ESCHERICHIA-COLI CELLS", BIOELECTROCHEMISTRY AND BIOENERGETICS, XX, XX, vol. 21, no. 1, 1 January 1989 (1989-01-01), pages 47-54, XP002467705, ISSN: 0302-4598, DOI: 10.1016/0302-4598(89)87005-9

<110> Samsung Electronics Co., Ltd.
<120> A method of amplifying a nucleic acid from blood
<130> EP49819FZ106pau
<160> 2
<170> KopatentIn 1.71
<210> 1
   <211> 20
   <212> DNA
   <213> Artificial sequence
<220>
   <223> forward primer
<400> 1
   agtgtggatt cggcactcct 20
<210> 2
   <211> 23
   <212> DNA
   <213> Artificial sequence
<220>
   <223> reverse primer
<400> 2
   gagttcttct tctaggggac ctg 23

## Description

### BACKGROUND OF THE INVENTION

### 1. Field of the Invention

The present invention relates to a method of amplifying a nucleic acid from a blood sample.

### 2. Description of the Related Art

In order to efficiently amplify or detect a target nucleic acid, first, the target nucleic acid needs to be separated from inhibiting materials of the sample that adversely affect a Polymerase Chain Reaction (PCR) of the sample. An example of inhibiting material is a red blood cell. Therefore, the amount of blood that can be directly used as PCR sample is limited. In general, if blood makes up to 1.5 % of the total reaction solution, PCR products cannot be obtained effectively.

In order to overcome this problem, a method of selectively destroying red blood cells may be used. U.S Patent No. 4,407,942 discloses a method of selectively destroying red blood cells using ammonium chloride. In addition, U.S. Patent No. 5,704,884 discloses a method including a) mixing a whole blood sample with a red blood cell destroying solution, wherein the destroying solution has a pH of 6 to 8 and includes ammonium chloride of 50 to 100 mM as well as a monocarboxylic acid of 0.01 to 0.1 parts by weight or a salt thereof; b) centrifuging the obtained mixture to form leukocyte pellets from the whole blood sample; c) removing the supernatant from the whole blood sample in order to wash the leukocyte pellets in a fresh destroying solution sample; and d) centrifuging the leukocyte pellets to separate them from the fresh destroying solution sample. All the operations a) through d) are performed within 20 minutes. In addition, U.S Patent No. 5,935,825 discloses a new PCR amplifying method in which a PCR is performed at a higher pH than the pH known in the conventional art.

Moreover, U. S Patent No. 6,284,117 discloses an apparatus and a method of reducing the ionic strength of a low volume solution used in the electronic transport of nucleic acid, protein and/or cells. The apparatus includes a tube shaped molecular weight cut-off membrane having a lumen, an ion-exchange resin, an electrodialysis electrode, and a chamber surrounding and housing the cut-off membrane, the ion-exchange resin, the electrodialysis electrode, wherein the cut-off membrane has inlet and outlet ports leading through the chamber and is embedded in the center of the ion-exchange resin. The chamber has inlet and outlet ports for exchanging a flowable material into and out of the chamber. The electrodialysis electrodes are positioned in a spaced axial alignment on opposite side of the chamber with respect to the membrane, and the method includes applying a current between the electrodialysis electrodes.

However, a method of using a large amount of blood directly in a PCR is not disclosed in the conventional art. Therefore, the inventors found that when electrodialysis is performed on a blood sample materials included in the sample that inhibit a PCR can be reduced, thus resulting in the present invention.

WO-A1-2005/083078 relates to a method and a device for extraction of biological materials from biological cells in a sample. The method of extracting biological material comprises the steps of: a) providing a sample chamber and a first and a second electrode, b) providing a liquid sample in the sample chamber, and c) exposing the liquid sample to an alternating electric field in said sample chamber so as to extract biological material from the biological cell. The method may furthermore comprise the step d) of performing analysis on a part of the exposed liquid sample.

### SUMMARY OF THE INVENTION

The present invention provides a method of efficiently amplifying a target nucleic acid from a sample, which is blood, using electrodialysis.

According to an aspect of the present invention, there is provided a method of amplifying a nucleic acid from a sample according to independent claim 1.

In one embodiment, applying an electric field to the sample can be performed by electrodialysis comprising: injecting the sample into a dilution compartment of an electrodialysis device, in which dilution compartment a molecular weight cut-off membrane constitutes, at least section wise, one or more walls of the dilution compartment; and applying a voltage generating an electric field to the sample in the dilution compartment and removing ionic materials from the dilution compartment.

In one embodiment, the diluting compartment includes 2 walls facing each other, which walls are formed, at least section wise, of the molecular weight cut-off membrane.

The molecular weight cut-off membrane may have the molecular weight cut-off of 1 to 500 kDa.

The voltage may be direct voltage (DC). The voltage applied during electrodialysis may be 10 to 200 V.

The electrodialysis may be performed for several seconds to several minutes, preferably for example for less than 200 seconds/1 ml blood sample. However, the period for performing the electrodialysis depends on the intensity of voltage and current and the volume of the solution, and a person skilled in the art is able to determine a suitable period for the performance based the above parameters.

In one embodiment, the method further comprises: applying an electric field to the blood sample by performing electrolysis comprising adding the sample to a cathode chamber of an electrolysis device and applying a voltage generating an electric field to the sample.

The voltage applied during electrolysis may be 1 to 100 V.

The electrolysis may be performed for several seconds to several minutes, for example less the 200 seconds/1 ml blood sample. However, the period for performing the electrolysis depends on the intensity of voltage and current and the volume of the solution, and a person skilled in the art is able to determine a suitable period for the performance based the above parameters.

The PCR may be performed using a reaction solution, wherein the amount of electrodialysed blood makes up 0.1 % to 30 % (v/v) of the reaction solution.

In one embodiment, electrolysis and electrodialysis may be performed on the sample before performing PCR. In a further embodiment, electrolysis is performed prior to or subsequent to performing electrodialysis.

### BRIEF DESCRIPTION OF THE DRAWINGS

The above and other features and advantages of the present invention will become more apparent by describing in detail exemplary embodiments thereof with reference to the attached drawings in which:
FIG. 1 is a diagram of an electrolysis device used according to an embodiment of the present invention;
FIG. 2 is a diagram showing the result of electrolysis when a voltage of 20 V and a current of 10 mA are applied for 40 seconds;
FIG. 3 is a diagram of an electrodialytic apparatus used according to an embodiment of the present invention;
FIG. 4 is a diagram showing the result after an electrodialysis is performed on blood by applying a voltage of 100 V and then, spinning-down the electrodialysed sample;
FIG. 5 is a diagram showing the result of a Polymerase Chain Reaction (PCR), wherein differently treated blood samples are used in a range of 0.5 % to 30 % (v/v) of the total volume of the reaction solution;
FIG. 6 is a diagram showing the result of a PCR, wherein differently treated blood samples are used in an amount of 3 % to 20 % (v/v) of the total volume of the reaction solution; and
FIG. 7 is a diagram showing PCR reaction solutions obtained after differently treated blood samples including 1000 *Escherichia coli* cells/glare adding in an amount of 3 % to 20 % (v/v) in respect to the volume of the reaction solution.

### DETAILED DESCRIPTION OF THE INVENTION

Hereinafter, the present invention will be described in more detail with reference to the accompanying drawings, in which exemplary embodiments of the invention are shown.

A method of amplifying a nucleic acid from a sample, which is blood, is provided, said method including the steps of performing an electrodialysis on the sample and reducing the ionic strength of the sample, and performing a Polymerase Chain Reaction (PCR) using the electrodialysed sample as a template.

The method of the present invention includes the operation of performing electrodialysis on a sample, e.g. a blood sample, to reduce the ionic strength of the sample. When the electrodialysis is performed on the blood sample, ionic materials included in blood are removed and thus, the ionic strength of the blood sample is reduced. Therefore, ionic materials that inhibit the PCR are removed, and preferably red blood cells are destroyed.

An electrodialytic or electrodialysis apparatus includes ion exchange membranes. These membranes facilitate specific ions to pass through the membranes under the influence of a polarized electric field. A fluid having a high concentration of undesired ions passes through a compartment of the apparatus, in which compartment ion exchange membranes are included in one or more of the compartment walls. "Electrodialysis" is well known in the field to which the present invention pertains and refers to a method, wherein a sample containing salt is placed between membranes and an electric field is applied thereto. The cations and anions of the sample migrate in opposite directions and pass through the membranes, leaving purer water between the membrane filters. The electrodialysis apparatus conventionally comprises three chambers defined by membranes. Two side chambers with electrodes have pure water. The cations and anions of the sample are transported through membrane from middle chamber to side chambers using electric field.

As the fluid passes through the compartment, ions are removed to an adjacent compartment. The compartment containing the fluid form which ions are removed is called a diluting (or dilution) compartment. The compartment into which the removed ions are transferred is called a concentrating compartment. In addition, when ion separation having low efficiency is sufficient for a process such as cell disruption, ion exchange membranes can be replaced with tube shaped molecular weight cut-off membranes. Therefore, the molecular weight cut-off membrane of the present invention includes an ion exchange membrane.

The operation of performing electrodialysis may include: injecting the blood sample into a diluting compartment in which molecular weight cut-off membranes constitute, at least section wise, one or more of the compartment walls; and applying a voltage to electrodes and generating an electric field to the sample and removing ionic materials from the diluting compartment.

As the molecular weight cut-off membrane, any material may be used, which is suited for freely transporting ionic materials having a low molecular weight, proteins, nucleic acids, and cells. Accordingly, the cut-off of the molecular weight cut-off membrane varies according to the materials to be removed. The cut-off of the molecular weight cut-off membrane is not particularly restricted and may have cut-off range of 1 kDa to 500 kDa. In addition, the molecular weight cut-off membrane may be formed of reproduced cellulose, polyethersulfone (PES), polysulfone (PS), or polyvinyl difluoride (PVDF) but is not limited thereto.

The voltage applied for performing electrodialysis according to an embodiment of the present invention is direct voltage and may be applied with the voltage of 10 to 200 V for several seconds to several minutes, for example than less 200 seconds/1 ml blood sample. However, the period for performing the electrodialysis depends on the intensity of voltage and current and the volume of the solution, and a person skilled in the art is able to determine a suitable period for the performance based the above parameters.

The method of the present invention includes the operation of performing PCR using the blood sample, on which electrodialysis was performed, as a template.

"PCR (polymerase chain reaction)" is well known in the field to which the present invention pertains. U.S. Patent Nos. 4,683,195, 4,683.202, and 4,965,188 disclose the PCR in detail. In the PCR, a primer is hybridized with a single strand of a target nucleic acid (also referred to as "template") under the presence of a polymerization agent such as DNA polymerase and dNTP. The DNA polymerase polymerizes the DNA strand that is complementary to the template strand. Once the primer extension products are denatured, a copy of the template is produced. Cycles of annealing, extending, and denaturalizing are performed for desired times in order to exponentially increase the amount of nucleic acid having the same sequence as the target nucleic acid.

The blood samples of the present invention, on which the electrodialysis is performed, can be used as the template of the PCR reaction without additionally extracting proteins. The PCR may be performed using 0.1 % to 30 % (v/v), for example, 0.5 % to 20 % (v/v) of electrodialysed blood sample as the template in a reaction solution.

The method of the present invention may further include performing electrolysis on the sample before performing the electrodialysis. Also, the method of the present invention may further include performing electrolysis on the electrodialysed sample, that is, after the electrodialysis is performed.

"Electrolysis" is well known in the field to which the present invention pertains and refers to a method comprising passing an electric current through a solution containing salts and generating ions by an oxidation and reduction reaction. In the electrolysis reaction, reduction reaction occurs at the cathode electrode and oxidation reaction occurs at the anode electrode. In the electrolysis, electrolyte solutions are added to each of a cathode chamber and an anode chamber separated by an ion-permeable membrane and a voltage is applied thereto. Then, the reaction occurs in an involuntary direction by current applied due to the voltage.

In the present invention, the electrolysis may be performed by adding the sample, either the initial sample prior to electrodialysis or the electrodialysed sample, into a cathode chamber and applying the voltage thereto. The voltage and the application time may be 1 V to 100 V and several seconds to several minutes, for example less than 200 seconds/1 ml blood sample, respectively, but are not limited thereto. However, the period for performing the electrolysis depends on the intensity of voltage and current and the volume of the solution, and a person skilled in the art is able to determine a suitable period for the performance based the above parameters. Due to the electrolysis, proteins included in the cathode chamber or the chamber in which the electrodialysis is performed, are denatured and lipid components of the membrane are dissolved. However, the present invention is not particularly restricted thereto.

In the method of amplifying a nucleic acid from blood of present invention, the electrolysis may be performed by adding the sample including blood or blood on which the electrodialysis is performed into a cathode chamber and applying voltage thereto. The voltage applied and the application duration may be 1 V to 100 V and several seconds to several minutes for example less than 200 seconds/1 ml blood sample, respectively, but are not limited thereto. However, the period for performing the electrolysis depends on the intensity of voltage and current and the volume of the solution, and a person skilled in the art is able to determine a suitable period for the performance based the above parameters

The present invention will be described in greater detail with reference to the following examples. The following examples are for illustrative purposes only and are not intended to limit the scope of the invention.

### Example 1: electrolysis of blood

In this example, the cathode chamber of an electrolysis device was filled with whole blood in order to perform the electrolysis thereon and the effects of electrolysis on the whole blood sample were observed.

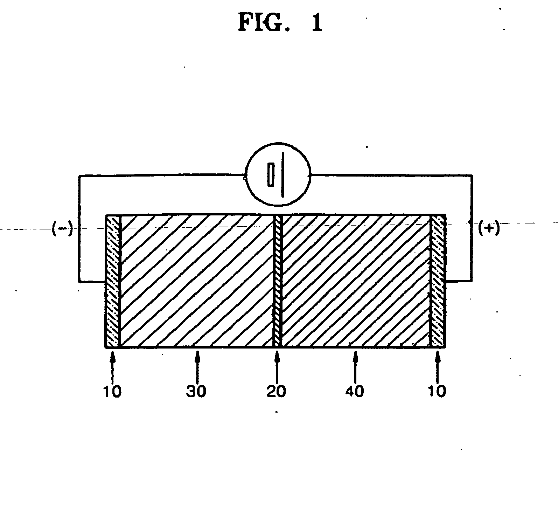

FIG. 1 is a diagram showing an electrolysis device used for performing electrolysis according to an embodiment of the present invention. Referring to FIG. 1, the device includes a cathode chamber 30, an anode chamber 40, and a cellophane film 20, wherein the cellophane film 20, that is, a molecular weight cut-off membrane, is interposed between the cathode chamber 30 and the anode chamber 40. The cathode chamber 30 and the anode chamber 40 are connected with a power source through electrodes 10, respectively. 5 ml of 300 mM Na₂SO₄ was filled in the anode chamber 40 as an electrolyte and 5 ml of human blood was filled in the cathode chamber 30.

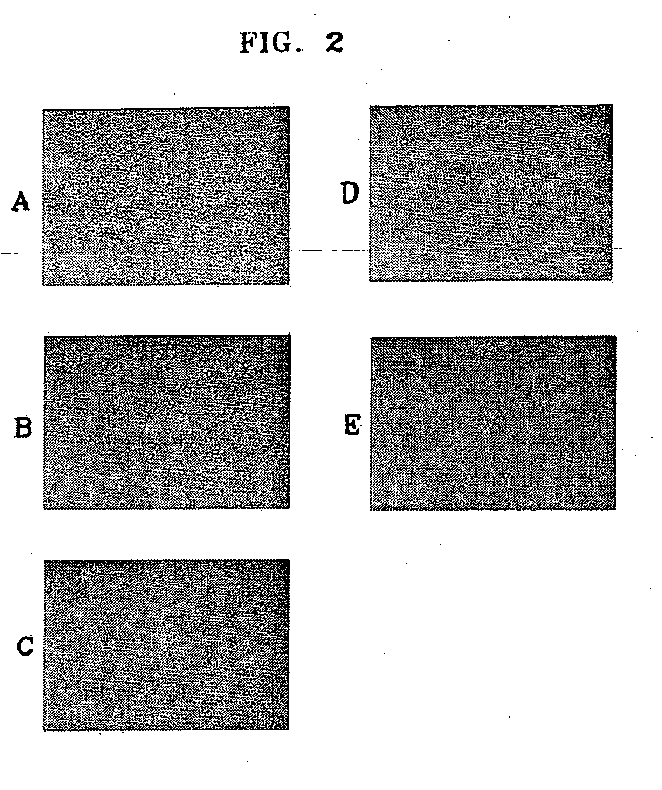

FIG. 2 is diagram showing the result when a voltage of 20 V and a current of 10 mA are applied for 40 seconds to the electrodes 10 by means of the power source (A:0 second, B:10 seconds, C:20 seconds, D:30 seconds, e:40 seconds). Referring to FIG. 2, as the electrolysis was performed on the sample, blood cells were disrupted and the blood color became the more transparent the longer the voltage was applied. The color change occurs due to denaturation of heme by the electrolysis, wherein the heme is originated from the red blood cells.

### Example 2: electrodialysis of blood

In this example, whole blood was filled in the diluting compartment of an electrodialysis device to perform the electrodialysis thereon and the effect of electrodialysis on the blood sample was observed.

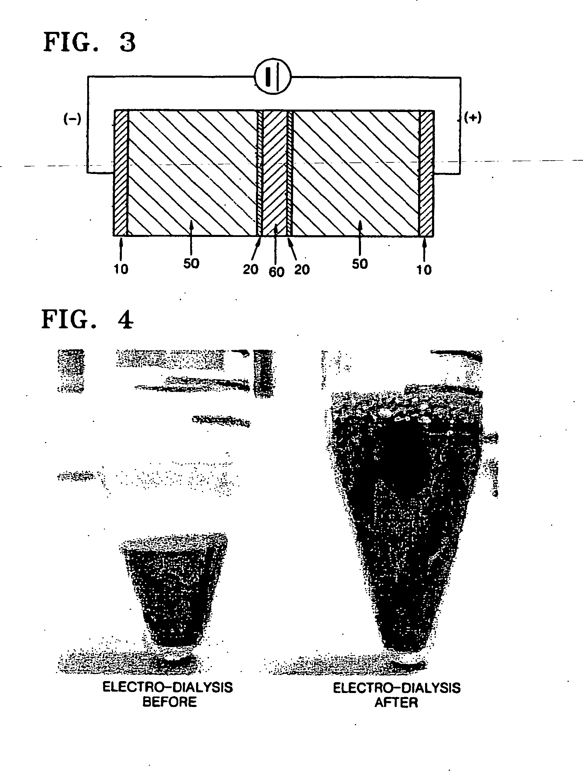

FIG. 3 is a diagram of an electrodialytic apparatus used for performing electrodialysis according to an embodiment of the present invention. Referring to FIG. 3, the apparatus includes a diluting compartment 60 in which two walls thereof opposing each other are formed of a cellophane film 20, that is a molecular weight cut-off membrane, and two concentrating compartments 50 are adjacent to the diluting compartment 60 and are separated from the dilution compartment 60 via the cellophane films 20. The concentrating compartments 50 are connected with the electrodes 10. The distance between the electrodes 10 was 6 cm and the area of the electrodes 10 was 80 cm². 200 ml of distilled water was filled in each of the concentrating compartments 50 and 5 ml of human blood used as sample was filled in the diluting compartment 60.

A voltage of 100 V was applied to the electrodes for 15 minutes, thereby generating an electric field to the sample. When the conductivity of the solutions in the concentrating compartments 50 significantly increased, an excessive amount of current flowed through the solutions and the sample in the concentration compartments 50 and the dilution compartment 60, respectively. In order to reduce the conductivity and control the maximum current at 100 mA or below, distilled water was circulated in the two concentrating compartments 50, i.e. the solution in the concentration compartments 50 was replaced with new distilled water when the current rose above 100 mA.

FIG. 4 is a diagram showing the result after electrodialysis was performed on the blood sample by applying a voltage of 100 V for 15 minutes and then, spinning-down the electrodialysed sample. Referring to FIG. 4, red blood cells were disrupted during the electrodialysis. Ionic materials were removed from the diluting compartment 60 into one of the concentrating compartment 50 so as to reduce the ionic strength of blood. As a result of the reduced ionic strength in the sample, red blood cells were disrupted, which can be seen by the fact that the heme of the electrodialysed sample is homogeneously dispersed in the solution rather than precipitated in intact red blood cells. Therefore, red blood cells can be disrupted using the electrodialysis without diluting a blood sample.

### Example 3: PCR using blood including Escherichia coli DNA as template

In this example, blood was treated using different methods and PCR was performed using different blood samples including *Escherichia coli* in a concentration of 1000 *Escherichia coli* cells/µℓ as a template.

The different blood samples used as PCR template were untreated blood (A), electrolyzed blood (B), electrodialysed blood (C), and blood on which the electrolysis was performed after electrodialysis (D). The device of Example 1 was used for performing the electrolysis. 2 ml of blood was filled into the cathode chamber 30 and 2 ml of Na₂SO₄ was filled into the anode chamber 40. Then a voltage of 50 V was applied to the electrodes for 15 minutes in order to have a maximum current of 30 mA.

The apparatus of Example 2 was used for performing electrodialysis. 5 ml of blood was filled in the diluting compartment 60 and 200 ml of distilled water was added to the two adjacent concentrating compartments 50. Then, a voltage of 100 V was applied to the electrodes 10. The electrolysis performed after the electrodialysis was performed was the same as described above.

The PCR was performed using different amounts of blood, which was treated as described above, as a template without extracting proteins. Primers having the nucleotide sequence of SEQ ID Nos: 1 and 2 (the target sequence was *Escherichia coli* genome DNA) are used for PCR and the conditions of the PCR are as follows.

The PCR was performed with an initial denaturation at 95 °C for 1 minute, a denaturation at 95 °C for 5 seconds, an annealing at 62 °C for 13 seconds, and extension at 72 °C for 15 seconds. The whole cycle of denaturing, annealing and extension was repeated 30 times. The final extension was performed at 72 °C for 1 minute. The composition of the reaction solution for PCR is illustrated in Table 1.

**Table 1.**

| Components (µl) | Sample 1 | Sample 2 | Sample 3 | Sample 4 | Sample 5 | Sample 6 |
|---|---|---|---|---|---|---|
| 1xbuffer | 5 | 5 | 5 | 5 | 5 | 5 |
| dNTP | 1 | 1 | 1 | 1 | 1 | 1 |
| Forward primer | 1 | 1 | 1 | 1 | 1 | 1 |
| Reverse primer | 1 | 1 | 1 | 1 | 1 | 1 |
| BSA | 5 | 5 | 5 | 5 | 5 | 5 |
| Taq polymerase | 0.5 | 0.5 | 0.5 | 0.5 | 0.5 | 0.5 |
| Distilled water | 35.25 | 35 | 34 | 33 | 30.5 | 20.5 |
| E.coli DNA | 1 | 1 | 1 | 1 | 1 | 1 |
| Sample | 0.25 | 0.5 | 1.5 | 2.5 | 5 | 15 |
| Total volume | 50 | 50 | 50 | 50 | 50 | 50 |
| Blood volume percent (%) | 0.5 | 1 | 3 | 5 | 10 | 30 |

The term "reaction solution" refers to the solution containing all components for PCR, which solution is placed in the reaction tube and subjected to the temperature treatment of PCR.

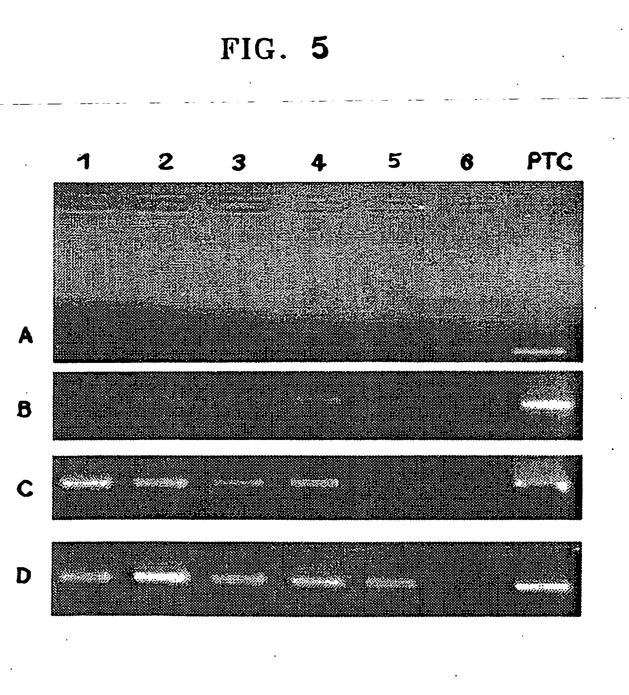

FIG. 5 is a diagram showing the result of the PCR in which respective blood samples 1 to 6 are used in a range of 0.5 % to 30 % (v/v) of the total volume of the PCR reactants (lane 1: 0.5%, lane 2: 1 %, lane 3: 3%, lane 4: 5%, lane 5: 10%, lane 6: 30%, lane PTC: positive control). Referring to FIG. 5, when blood was added in an amount of 0.5 % of the total volume of the PCR reactants, PCR products were obtained and when the blood was added in an amount above 0.5 % (v/v) of the total volume of the PCR reactants, PCR products were not obtained (A). In addition, when the blood samples 1 to 6 were treated by electrolysis (B) or electrodialysis (C), respectively and were even added in an amount of up to 5 % (v/v) of the PCR reactants, PCR products were obtained. When the blood sample was treated by electrodialysis and subsequently by electrolysis, even when the treated sample was in an amount of up to 10 % (v/v) of the PCR reactants, PCR products were obtained (D). When untreated blood (A) was used in an amount of up to 30 % (v/v) of the PCR reactants, blood solidified during the PCR, however, if blood was treated by one of electrolysis and electrodialysis, blood was not solidified (not illustrated).

### Example 4: PCR using blood including Escherichia coli as template

In this example, blood including *Escherichia coli* was treated using different methods and PCR was performed using the treated blood as a template.

The blood used as a template was untreated blood (C), electrolyzed blood (B), and blood on which electrolysis was performed after electrodialysis (A). The blood sample contained *Escherichia coli* in a concentration of 1,000 *Escherichia coli* cells/µℓ, 100 *Escherichia coli cells*/*µℓ,* and 10 *Escherichia coli* cetts/µℓ, respectively.

The device of Example 1 was used for performing the electrolysis. 2 ml of blood was filled in the cathode chamber 30 and 2 ml of Na₂SO₄ was filled in the anode chamber 40. Then a voltage of 50 V was applied to the electrodes for 15 minutes so as to have a maximum current of 30 mA. The apparatus of Example 2 was used for performing the electrodialysis. 5 ml of blood was filled into the diluting compartment 60 and 200 ml of distilled water was added to the two adjacent concentrating compartments 50. Then, a voltage of 100 V was applied to the electrodes 10. The electrolysis performed after the electrodialysis was performed was the same as described above.

The PCR was performed using different amounts of blood, which was treated as described above, as a template without extracting proteins. Primers having the nucleotide sequence of SEQ ID Nos: 1 and 2 (the target sequence was *Escherichia coli* genome DNA) are used for PCR and the conditions of the PCR are as follows.

The PCR was performed with an initial denaturation at 95 °C for 1 minute, a denaturation at 95°C for 5 seconds, an annealing at 62 °C for 13 seconds, and extension at 72 °C for 15 seconds. The cycle of denaturing, annealing and extension was repeated 30 times. The final extension was performed at 72 °C for 1 minute. The composition of the PCR reactants is illustrated in Table 2.

**Table 2.**

| Components (µl) | Sample 1 | sample 2 | Sample 3 | Sample 4 |
|---|---|---|---|---|
| 1xbuffer | 5 | 5 | 5 | 5 |
| dNTP | 1 | 1 | 1 | 1 |
| Forward primer | 1 | 1 | 1 | 1 |
| Reverse primer | 1 | 1 | 1 | 1 |
| BSA | 5 | 5 | 5 | 5 |
| Taq polymerase | 0.5 | 0.5 | 0.5 | 0.5 |
| Distilled water | 35 | 34 | 31.5 | 26.5 |
| Sample | 1.5 | 2.5 | 5 | 10 |
| Total volume | 50 | 50 | 50 | 50 |
| Blood volume percent (%) | 3 | 5 | 10 | 20 |

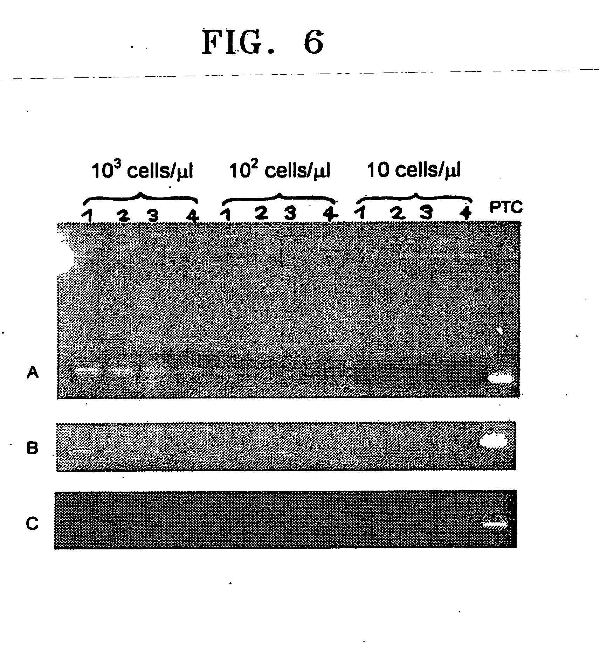

FIG. 6 is a diagram showing the result of the PCR in which respective blood samples 1 to 4 are used in an amount of 3 % to 20 % (v/v) of the total volume of the PCR reactants (lane 1: 3%, lane 2: 5%, lane 3: 10%, lane 4: 20%, PTC: positive control). Referring to FIG. 6, when untreated blood was added in an amount of 3 % (v/v) of the total volume of the PCR reactants, irrespective of the concentration of *Escherichia coli,* PCR products were not obtained (C). The PCR does not occur due to inhibitory materials included in blood that adversely affect PCR. In addition, when blood samples having a concentration of 100 *Escherichia coli* cells/µℓ and 1,000 *Escherichia coli* cells/µℓ were treated by electrolysis only and were added in an amount of up to 3 % (v/v) and 10 % (v/v) of the PCR reactants, respectively, PCR products can be obtained (B). Moreover, when blood samples having a concentration of 100 *Escherichia coli* cells/µℓ and 1,000 *Escherichia coli* cells/µl were treated by electrodialysis and subsequently by electrolysis and were added in an amount of up to 5 % (v/v) and 20 % (v/v) of the PCR reactants, respectively, PCR products can be obtained (A). These results indicate that, applying the methods of the present invention, PCR is possible using a PCR sample having a higher concentration of blood than using the initial blood as PCR sample.

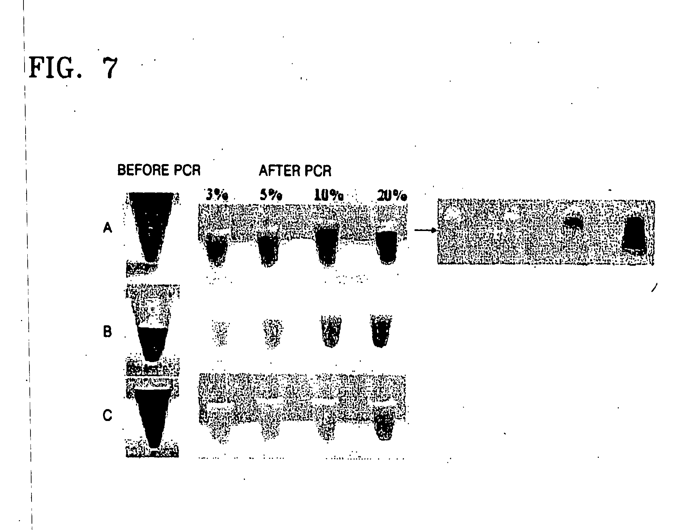

FIG. 7 is a diagram showing PCR reaction solutions obtained after blood samples including Escherichia *coli in* a concentration of 1000 Escherichia coli cells/µl are treated according to various methods defined below by adding an amount of 3 to 20 % (v/v) of the PCR reaction solutions into the PCR reaction solutions.

Referring to FIG. 7, when untreated blood was added in an amount of 3 % (v/v) or above and up to 20 % (v/v) to the PCR reaction solutions, respectively, the color of the solution darkened and the blood solidified, respectively (A). The color got lighter in blood treated by the electrolysis (B), and the color got even lighter than the blood treated by the electrolysis, when the blood was treated by electrolysis after electrodialysis (C).

As described above, when blood is treated by at least one of the electrolysis and electrodialysis, the performance of PCR significantly improves. In the method of amplifying a nucleic acid from a sample, such as a body fluid like blood, according to an embodiment of the present invention, a large amount of blood sample can be used in the PCR reaction solution as a template and thus, the amplification efficiency can be increased. Therefore, a target nucleic acid included in blood, for example, bacteria or virus, can be efficiently detected.
## Claims

1. A method of amplifying a nucleic acid from a sample, which is blood, comprising:
applying an electric field to the sample by performing electrodialysis on the blood to reduce the ionic strength of the sample to disrupt red blood cells; and subsequently performing a Polymerase Chain Reaction (PCR) using the electrodialysed blood as a template.

2. The method of claim 1, wherein applying an electric field to the blood is performed by electrodialysis comprises: injecting the blood into a dilution compartment (60) of an electrodialysis device, in which dilution compartment (60) a molecular weight cut-off membrane (20) constitutes, at least section wise, one or more walls of the dilution compartment (60); and
applying a voltage generating an electric field to the blood in the dilution compartment and removing ionic materials from the dilution compartment (60).

3. The method of claim 2 wherein the molecular weight cut-off membrane (20) has the molecular weight cut-off of 1 kDa to 500 kDa.

4. The method of claim 2 or 3, wherein the diluting compartment (60) includes 2 walls facing each other, which walls are formed, at least section wise, of the molecular weight cut-off membrane (20).

5. The method of any of claims 2 to 4, wherein a direct voltage is applied.

6. The method of any of claims 2 to 5, wherein a voltage of 10 V to 200 V is applied during electrodialysis.

7. The method of any of claims 2 to 6, wherein the electrodialysis is performed for 15 minutes or less.

8. The method of any of claims 2 to 6, wherein the electrodialysis is performed for less than 200 seconds/1 ml blood.

9. The method of claim 1, further comprising: applying an electric field to the blood by performing electrolysis comprising adding the blood to a cathode chamber of an electrolysis device and applying a voltage generating an electric field to the blood.

10. The method of claim 9, wherein a voltage of 1 V to 100 V is applied during electrolysis.

11. The method of claim 9 or 10, wherein electrolysis is performed for 15 minutes or less.

12. The method of claim 9 or 10, wherein the electrolysis is performed for less than 200 seconds/1 ml blood.

13. The method of any of claims 1 to 12, wherein PCR is performed using a reaction solution, wherein the amount of electrodialysed blood makes up 0.1 % to 30 % (v/v) of the reaction solution.

14. The method of any of claims 1 to 13, comprising performing electrolysis and electrodialysis on the blood before performing PCR.

15. The method of claim 14, wherein electrolysis is performed prior to or subsequent to performing electrodialysis.

## Patentansprüche

1. Verfahren zum Amplifizieren einer Nukleinsäure aus einer Probe, bei der es sich um Blut handelt, umfassend:
das Anlegen eines elektrischen Feldes an die Probe durch das Durchführen einer Elektrodialyse an dem Blut, um die Ionenstärke der Probe zu verringern, um rote Blutzellen aufzubrechen; und anschließend das Durchführen einer Polymerase-Kettenreaktion (PCR) unter Verwendung des elektrodialysierten Blutes als Matrize.

2. Verfahren nach Anspruch 1, wobei das Anlegen eines elektrischen Feldes an das Blut durchgeführt wird durch Elektrodialyse umfassend: das Injizieren des Blutes in eine Verdünnungskammer (60) einer Elektrodialysevorrichtung, wobei in dieser Verdünnungskammer (60) eine Molekulargewichtsausschlussmembran (20) zumindest abschnittsweise eine oder mehrere Wände der Verdünnungskammer (60) darstellt; und
das Anlegen einer Spannung, die ein elektrisches Feld erzeugt, an das Blut in der Verdünnungskammer und das Entfernen von ionischen Materialien aus der Verdünnungskammer (60).

3. Verfahren nach Anspruch 2, wobei die Molekulargewichtsausschlussmembran (20) einen Molekulargewichtsausschluss von 1 kDa bis 500 kDa aufweist.

4. Verfahren nach Anspruch 2 oder 3, wobei die Verdünnungskammer (60) zwei einander zugewandte Wände einschließt, wobei diese Wände zumindest abschnittsweise aus der Molekulargewichtsausschlussmembran (20) gebildet sind.

5. Verfahren nach einem der Ansprüche 2 bis 4, wobei eine Gleichspannung angelegt wird.

6. Verfahren nach einem der Ansprüche 2 bis 5, wobei eine Spannung von 10 V bis 200 V während der Elektrodialyse angelegt wird.

7. Verfahren nach einem der Ansprüche 2 bis 6, wobei die Elektrodialyse während 15 Minuten oder weniger durchgeführt wird.

8. Verfahren nach einem der Ansprüche 2 bis 6, wobei die Elektrodialyse während weniger als 200 Sekunden/1 ml Blut durchgeführt wird.

9. Verfahren nach Anspruch 1, außerdem umfassend: das Anlegen eines elektrischen Feldes an das Blut durch das Durchführen einer Elektrolyse umfassend das Zugeben des Blutes zu einer Kathodenkammer einer Elektrolysevorrichtung und das Anlegen einer Spannung, die ein elektrisches Feld erzeugt, an das Blut.

10. Verfahren nach Anspruch 9, wobei eine Spannung von 1 V bis 100 V während der Elektrolyse angelegt wird.

11. Verfahren nach Anspruch 9 oder 10, wobei die Elektrolyse während 15 Minuten oder weniger durchgeführt wird.

12. Verfahren nach Anspruch 9 oder 10, wobei die Elektrolyse während weniger als 200 Sekunden/1 ml Blut durchgeführt wird.

13. Verfahren nach einem der Ansprüche 1 bis 12, wobei eine PCR durchgeführt wird unter Verwendung einer Reaktionslösung, wobei die Menge an elektrodialysiertem Blut 0,1 % bis 30 % (Vol./Vol.) der Reaktionslösung ausmacht.

14. Verfahren nach einem der Ansprüche 1 bis 13, umfassend das Durchführen einer Elektrolyse und Elektrodialyse an dem Blut vor dem Durchführen einer PCR.

15. Verfahren nach Anspruch 14, wobei eine Elektrolyse vor oder nach dem Durchführen einer Elektrodialyse durchgeführt wird.

## Revendications

1. Procédé d'amplification d'un acide nucléique à partir d'un échantillon, qui est du sang, comprenant : l'application d'un champ électrique sur l'échantillon par exécution d'une électrodialyse sur le sang afin de réduire la force ionique de l'échantillon pour dissocier les globules rouges ; et ensuite l'exécution d'une réaction en chaîne de la polymérase (PCR) à l'aide du sang électrodialysé en tant que matrice.

2. Procédé selon la revendication 1, dans lequel l'application d'un champ électrique sur le sang qui est exécutée par électrodialyse comprend : l'injection du sang dans un compartiment de dilution (60) d'un dispositif d'électrodialyse, où dans ce compartiment de dilution (60) une membrane à seuil de coupure de masse moléculaire (20) constitue, au moins par tronçons, une ou plusieurs parois du compartiment de dilution (60) ; et
l'application d'une tension générant un champ électrique sur le sang dans le compartiment de dilution et l'élimination des matières ioniques du compartiment de dilution (60).

3. Procédé selon la revendication 2 dans lequel la membrane à seuil de coupure de masse moléculaire (20) a un seuil de coupure de poids moléculaire de 1 kDa à 500 kDa.

4. Procédé selon la revendication 2 ou 3, dans lequel le compartiment de dilution (60) inclut 2 parois qui se font face, lesdites parois étant formées, au moins par tronçons, de la membrane à seuil de coupure de masse moléculaire (20).

5. Procédé selon n'importe lesquelles des revendications 2 à 4, dans lequel une tension continue est appliquée.

6. Procédé selon n'importe lesquelles des revendications 2 à 5, dans lequel une tension de 10 V à 200 V est appliquée pendant l'électrodialyse.

7. Procédé selon n'importe lesquelles des revendications 2 à 6, dans lequel l'électrodialyse est exécutée pendant 15 minutes ou moins.

8. Procédé selon n'importe lesquelles des revendications 2 à 6, dans lequel l'électrodialyse est exécutée pendant moins de 200 secondes/1 ml de sang.

9. Procédé selon la revendication 1, comprenant en outre : l'application d'un champ électrique sur le sang par exécution d'une électrolyse comprenant l'ajout du sang dans une chambre cathodique d'un dispositif d'électrolyse et l'application d'une tension générant un champ électrique sur le sang.

10. Procédé selon la revendication 9, dans lequel une tension de 1 V à 100 V est appliquée pendant l'électrolyse.

11. Procédé selon la revendication 9 ou 10, dans lequel l'électrolyse est exécutée pendant 15 minutes ou moins.

12. Procédé selon la revendication 9 ou 10, dans lequel l'électrolyse est exécutée pendant moins de 200 secondes/1 ml de sang.

13. Procédé selon n'importe lesquelles des revendications 1 à 12, dans lequel la PCR est exécutée à l'aide d'une solution réactionnelle, où la quantité de sang électrodialysé constitue 0,1 % à 30 % (v/v) de la solution réactionnelle.

14. Procédé selon n'importe lesquelles des revendications 1 à 13, comprenant l'exécution d'une électrolyse et d'une électrodialyse sur le sang avant l'exécution de la PCR.

15. Procédé selon la revendication 14, dans lequel l'électrolyse est exécutée avant ou après l'exécution de l'électrodialyse.
